# EUROPEAN PATENT SPECIFICATION

(11) **EP 1 682 097 B1**
(45) Date of publication and mention of the grant of the patent: **22.12.2010**
(21) Application number: 04797734.3
(22) Date of filing: 09.11.2004
(51) Int. Cl.: A61K 9/20

(54) **NON-TABLETTED, CHEWABLE, INDIVIDUALLY DOSED ADMINISTRATION FORMS**
NICHT TABLETTENFÖRMIGE KAUBARE EINZELN DOSIERTE VERABREICHUNGSFORMEN
FORMES POSOLOGIQUES INDIVIDUELLES MASTICABLES PAS EN COMPRIMES

(30) Priority: 10.11.2003 ES 200302612
(43) Date of publication of application: 26.07.2006
(73) Proprietor: Almirall, S.A., 08022 Barcelona (ES)
(72) Inventor: FABREGAS VIDAL, José Luis, E-08015 Barcelona (ES); MASSO CARBONELL, Antoni, E-08800 Vilanova i la Geltru(Barcelona) (ES); GARCIA GONZALEZ, Nuria, E-08025 Barcelona (ES); GUIRO COLL, Pere, E-08030 Barcelona (ES)
(74) Representative: Srinivasan, Ravi Chandran
(86) International application number: PCT/EP2004/012658
(87) International publication number: WO 2005/048974

(56) References cited:
- WO-A-02/064109
- US-A- 4 761 407
- US-A- 6 060 078

## Description

The invention relates to individually dosed administration forms for pharmaceutically active compounds, consisting of non-tabletted, chewable gel compositions packaged in blisters or cavities; to a process for the manufacture of such individually dosed administration forms; to individually dosed administration forms obtainable by the above-mentioned process; and to the use of a stabilising agent to enhance the ease of removal of the composition from the blisters or cavities.

Chewable delivery systems, such as chewing gums, are highly desirable means for the oral administration of pharmaceutically active compounds. A disadvantage of chewing gum compositions is that they generally include a water insoluble gum base, which remains in the mouth and must be disposed of. In addition, many active compounds may have affinity for the gum base, making thus accurate dosing difficult.

British Patent application GB 2 009 597 discloses chewable and swallowable, gelled antacid compositions. The compositions are obtained by dispersing an antacid in a solution comprising water, a carbohydrate or a polyhydric alcohol as a bodying agent and an amount of gelling agent sufficient to cause the liquid dispersion to set to a self-supporting gel after cooling. In a preferred embodiment the still liquid dispersion can be poured before cooling into oral unit dosage moulds and allowed to set

This process no longer requires separate shaping and packaging of solid administration forms. These are given their particular shape during the packaging operation by simple application of the softened composition into a substrate with the desired shape, followed by solidification. This results in an improved cost efficiency of the overall manufacturing process.

International patent application WO 87/00429 describes opacified gelatine compositions and processes for their manufacture. The compositions comprise fats, fatty oils or fat derivatives to improve the light stability of the dyes used to oolour the gelatine compositions. The specification states that all fats, fatty oils or fat derivatives of synthetic or natural origin, as well as partially hydrogenated products can be used, provided that they are physiologically safe.

US-A-4761407 discloses an "instantaneous solidified oral form" which can be produced by a continuous process. The only gelatin based formulations disclosed in this document contain only small amounts of glycerol monostearate.

It has now been found by the inventors that the use of gelatine as a gelling agent for the manufacture of non-tabletted, chewable compositions as those described in the prior art yields compositions that, upon ageing, do often present the problem that they cannot be easily removed from the packaging where they have been shaped without leaving residues in the packaging. The problem of residues left in the packaging upon removal of the jelly composition is particularly pronounced for compositions comprising a high amount of alkaline ingredients since these ingredients tend to destabilize the gelatine matrix. The problem is also particularly pronounced when the shape of the packaging shows edges or portions with a small radius of curvature.

The inventors have solved this problem by incorporating into a matrix material, comprising a mixture comprising a gelatine, at least one water-soluble alcohol and/or water as a solvent and at least one stabilising agent selected from the group consisting of esters of glycerine and fatty acids and products resulting from the alcoholysis / esterification reaction of such esters of glycerine and fatty acids with polyethyleneglycols, the stabilising agent having a melting point in the range of 42° C to 63° C. This results in non-tabletted, chewable, individually dosed administration forms comprising a composition of at least one pharmaceutically active substance dissolved or dispersed within the matrix material, which composition is plastic at elevated temperature. These administration forms can be removed from the packaging without leaving residues.

Accordingly, the present invention provides a non-tabletted, chewable, individually dosed administration form packaged in a blister or cavity shaped from a film, which administration form is a composition of at least one pharmaceutically active substance dissolved or dispersed within a matrix material comprising a mixture of at least 0.2% by weight, based on the composition, of a gelatine, an amount greater than 1 % by weight, based on the composition, of at least one stabilising agent, and at least one water-soluble alcohol and/or water as a solvent, which composition is plastic at elevated temperature, **characterised in that**
a) the stabilising agent is selected from the group consisting of (i) esters of glycerine and fatty acids; (ii) products resulting from the alcoholysis / esterification reaction of such esters of glycerine and fatty acids with polyethyleneglycols; and
b) in that the stabilising agent has a melting point in the range of 42° C to 63° C
c) in that water is present in an amount not greater than 46% by weight of the composition.

In a preferred embodiment of the present invention only one stabilising agent is incorporated into the matrix material.

As essential ingredients the composition of the present invention comprises at least one pharmaceutically active substance, gelatine present in an amount of at least 0,2% by weight of the composition, at least one stabilising agent as described above present in an amount greater than 1% by weight of the formulation, and at least one water-soluble alcohol and/or water as a solvent, wherein water is present in an amount not greater than 46% by weight of the composition. It may also comprise bodying agents that impart texture and body to the final gel, and other optional components such as preservatives, antioxidants, defoaming agents, sweeteners, taste-masking agents, colour and flavours. It is a preferred embodiment of the present invention that only one stabilising agent is used.

The bodying agents suitable for the present invention are sugars such as glucose, sucrose and fructose, sugar alcohols such as sorbitol, mannitol and maltitol and polysaccharides such as starch, cellulose and functionalised cellulose derivatives.

To ensure consumer acceptability it is preferred that the non-tabletted, individually dosed administration forms of the present invention have compositions showing no plastic deformation at temperatures below 37°C.

Gelatine is a protein obtained by extraction from animal raw materials containing collagen such as skins and bones, which have been previously conditioned by acidic or alkaline treatment. Commercially available gelatine typically contains 84-92% protein, 0,1-2% salts and the rest is water.

Commercially available gelatines are classified according to the raw material from which they have been obtained and according to their ability to gel, which is customarily measured as Bloom gel strength.

Although all types of gelatine can be used for the manufacture of the individually dosed administration forms of the present invention, it has been found that gelatines with a Bloom range comprised between 140 and 270 degrees Bloom, preferably between 180 and 250 degrees Bloom yield composition with optimum consumer acceptance in terms of palatability. Gelatines obtained though alkaline treatment are in general preferred to those obtained through acidic treatment.

The compositions of the present invention comprise gelatine in an amount greater than 0,2% by weight of the composition, more preferably greater than 1% by weight and still more preferably greater than 5% by weight of the composition.

The stabilising agent of the present invention is selected from the group consisting of esters of glycerine and fatty acids and products resulting from the alcoholysis / esterification reaction of such esters of glycerine and fatty acids with polyethyleneglycols having a melting point in the range of 42° C to 63° C.

Examples of such stabilising agents are the mono-, di- and triesters of glycerine with fatty acids and mixtures thereof, preferably the diesters of glycerine with fatty acids. Preferred fatty acids are those selected from C10-C20, preferably C16-C18, unsaturated, saturated fatty acids. Examples of such fatty acids are lauric, oleic, linoleic, linolenic, palmitic and stearic acids. An example of a preferred commercially available ester is Estol ® 3745 GDS T2 from Uniqema. Other examples of stabilising agents are the products of the alcoholysis/esterification reaction of the esters of glycerine and fatty acids mentioned above. Preferred examples are products of the alcoholysis/esterification reaction of hydrogenated palm kernel oil or hydrogenated palm oil with PEG 1500, such as Gelucire ® 44/14 and Gelucire ® 50/13 from Gattefossé.

In an embodiment of the invention the solvent or solvents present in the composition is/are used in a total amount of at least 10% by weight, more preferably greater than 25% by weight still more preferably greater than 50% by weight of the composition.

In a preferred embodiment of the invention the composition comprises more than 46% by weight of the composition of at least one water-soluble alcohol.

The amount of water in the compositions of the invention is not greater than 46% by weight, preferably not greater than 35% by weight, most preferably not greater than 25% by weight, most preferably not greater than 15% by weight of the composition.

The compositions of the present invention comprise at least one pharmaceutically active substance which is dispersed or dissolved within the matrix material when it is in the molten state. The pharmaceutically active substance need not be in any specific form for its successful incorporation within the molten matrix material, in particular it is not required, and also not preferred, that the pharmaceutically active substance is provided as a component of a shearform matrix carrier prepared by flashflow processing.

In an embodiment of the present invention the non-tabletted, individually dosed administration forms comprise more than 18% by weight of a pharmaceutically active substance.

Suitable pharmaceutically active substances that may be contained in the individually dosed administration forms of the present invention vary widely and generally represent any stable drug combination. Illustrative categories and specific examples include:
a) ANTACIDS:
   i) Inorganic or organic salts of aluminium, for example, aluminium allantoinate, aluminium aminoacetate, aluminium phosphate, aluminium silicate, aluminium glucoheptanoate or aluminium polygalacturonate.
   ii) Inorganic or organic salts of bismuth, for example, bismuth aluminate, bismuth carbonate, bismuth silicate, bismuth subcarbonate or bismuth citrate.
   iii) Inorganic or organic salts of calcium, for example, calcium phosphate or calcium aminoacetate.
   iv) Inorganic or organic salts of magnesium, for example, magnesium carbonate, basic magnesium carbonate, magnesium phosphate or magnesium silicate.
   v) Oxides and hydroxides, such as aluminium oxide, algeldrate (aluminium hydroxide), magnesium or calcium oxides or hydroxides.
   vi) Mixed salts of aluminium and sodium as silicate, mixed salts of aluminium and magnesium as hydrotalcite (basic aluminium and magnesium carbonate), almagate (basic aluminium and magnesium carbonate) or magaldrate (basic aluminium and magnesium sulphate), mixed salts of bismuth and magnesium as magnesium silicate, and magnesium aluminosilicates, as simaldrate or almasilate.
   vii) Hydrogen carbonates as sodium or potassium hydrogen carbonates.
   viii) Glycine.
   ix) Alginic acid and salts thereof.
   and mixtures thereof.
b) DRUGS FOR PEPTIC ULCER AND GASTRO-OESOPHAGEAL REFLUX Ranitidine*, Nizatidine, Famotidine, Cimetidine, Roxatidine, Pifatidine, Roxatidine, Sufotidine, Lafutidine, Osutidine, Pantoprazole, Omeprazole, Lansoprazole, Esomeprazole, Rabeprazole, Esaprazole, Pariprazole, Aripiprazole, Leminoprazole, Amoxicillin, Trospectomycin, Clarithromycin, Zinc Acexamate, Cetraxate, Rotraxate, Dosmalfate, Flavalfate, Sucralfate, Bismuth salts as bismuth citrate or subsalicylate, Triletide, Dicloguamine, Sulfoxazine, Rioprostil, Ritipenem, Trimoprostil, Benexate, Pramipide, Misoprostol, Alaptide, Proglumide, Azuletil, Trepenone, Polyenephosphatidylcholine, Plaunotol, Troxipide, Midoriamine, Ecabet, Quinotolast, Sulglicotide, Nitazoxanide, Revaprazan, and mixtures thereof.
c) DRUGS FOR FUNCTIONAL GASTROINTESTINAL DISORDERS; PROPULSIVES
   Metoclopramide, Cinitapride, Clebopride, Cisapride, Zacopride, Mosapride, Itopride, Prucalopride, Domperidone, Ecabapide, Polycarbophil calcium, Tegaserod, and mixtures thereof.
d) LAXATIVES
   Sennatin, Sennosides A + B, Glycerol, Picosulfate, Lactitol, Bisacodyl, Polyethylene glycol, Lactulose, Basic magnesium carbonate, and mixtures thereof.
e) ANTIOBESITY PRODUCTS
   Orlistat, Amfebutamone, Bupropion, Diethylpropion, Sibutramine, Fluoxetine, Metaraminol, Mazindol, Chorionic gonadotrophin, Phentermine, Metamfetamine, Phendimetrazine, Benzfetamine, Phenylpropanolamine, Fenproporex, and mixtures thereof.
f) DIGESTIVES; ENZIME PREPARATIONS
   Amilase, Cellulase, Lactase, Lipase, and mixtures thereof.
g) VITAMINES
   Mixtures of vitamines, mixtures of oligoelements, and mixtures thereof.
h) APPETITE STIMULANTS
   Pizotifen, Cryptoheptadine, Carnitine, Stolimine, and mixtures thereof.
i) ANTITHROMBOTIC AGENTS; PLATELET AGGREGATION INHIBITORS
   Ditazole, Acetylsalicylic acid, Trifusal, Epoprostenol, Eptifibatide, Heparin, Clopidrogel, Dipyridamole, Abciximab, Ticlopirine, Dalteparin, Danaparoid, Warfarin, Phenindione, Dicoumarol, Epoprostenol, Enoxaparin, Nadroparin, Antithrombin III, Indobufen, Parnaparin, Tinzaparin, Dermatan, Desirudin, Reviparin, Thombomoduline, Bivalirudin, Ardeparin, Lepirudin, Tifacogin, Fondaparine, Fenprocumone, Certoparin, Bemiparin, Idraparinux, Acenocoumarol, Gabexate, Sulodexide, Defibrotide, Isbogrel, Cilostazol, Ciprostene, Ataprost, Sulotroban, Taprostene, Cloricromen, Picotamide, Alprostadil, Sulfinpyrazone, Beraprost, Daltroban, Variprost, Satrigel, Sarpogrelate, Tirofiban, Ecraprost, Lamifiban, Lefradafiban, Xemilofiban, Polycosinol, Roxifiban, Lotrafiban, Sibrafiban, Alnidofibatide, Orbofiban, Argatroban, Ticlomarol, and mixtures thereof.
j) ANTIANEMIC PREPARATIONS; TRIVALENT IRON PREPARATIONS
   Ferritine, Ferric proteine succinate, Ferric dextran and mixtures thereof.
k) ANTIARRHYTHMICS
   Quinidine, Esmolol, Pirmenol, Acecainide, Pilsicainide, Recainam, Penticainide, Flecainide, Adenosine, Lidocain, Metoprolol, Propranolol, Nadolol, Oxprenolol, Phenytoin, Acebutolol, Sotalol, Carteolol, Medigoxine, Procainamide, Bretylium, Amiodarone, Disopyramide, Mexiletine, Moracizine, Tocainide, Propafenone, Barucainide, Alprenolol, Otenzepad, Verapamil, Diprafenone, Etacizin, Bidisomide, Arotinolol, Cibenzoline, Tiracizine, Pindolol, Diltiazem, Atenolol, Dofetilide, Tedisamil, Sematilide, Sotalol, Almokalant, Nifekalant, Ibutilide, Landiolol, Dronedarone, Talinolol, Tecadenoson, Digoxin, Indenolol, Prajmalium, Aprindine, Bunaftine, Butobendine, Lorajmine, Lorcainide, and mixtures thereof.
l) CARDIAC STIMULANTS, ORGANIC NITRATES
   Isosorbide mononitrate or dinitrate, Nitroglycerol, Pentaerythrityl tetranitrate, Molsidomine, and mixtures thereof.
m) ANTIHYPERTENSIVES; ALPHA ADRENORECEPTOR ANTAGONISTS
   Doxazosin, Urapidil, Nipradilol, Indoramin, Prazosin, Labetalol, Amosulalol, Terazosin, Monatepil, and mixtures thereof.
n) DIURETICS
   Triamterene, Canrenoate, Spironolactone, Furosemide, Torasemide, Cicletanine, Piretanide, Chlorothiazide, Chlortalidone, Hydroflumethiazide, Bendroflumethiazide, Methyclothiazide, Polythiazide, Clopamide, Quinethazone, Bumetanide, Indapamide, Xipamide, Cyclopenthiazide, Canrenone, Docarpamine, Hydrochlorothiazide, Metolazone, Azosemide, Anaritide, Ularitide, Ecadotril, Candoxatril, Amiloride, Ethacrynic acid, Conivaptan, Telmisartan, Mebutizide, and mixtures thereof.
o) PERIPHERAL VASODILATORS
   Dihydroergocristine, Piracetam, Nicergoline, Vinburnine, Cadralazine, Flunarizine, Metergoline, Hydralazine, Fasudil, Nicorandil, Linsidomine, Sildenafil, Cinnarizine, Heptaminol, Almitrine, Raubasine, Pentoxifyline, Trimetazidine, Buflomedil, Alprostadil, Brovincamine, Cinepazet, Dilazep, Lidoflazine, Molsidomine, Nicorandil, Nifedipine, Trapidil, Viskenit, and mixtures thereof.
p) VASOPROTECTIVES
   Diosmin, Hidroxmin, Hesperidin, Troxerutin, and mixtures thereof.
q) ANTI HYPERTENSIVES - SELECTIVE BETA BLOCKING AGENTS
   Atenolol, Esmolol, Carteolol, Metoprolol, Bisoprolol, Carvedilol, Nebivolol, Propranolol, Tertatolol, Betaxolol, Cetamolol, Nipradilol, Tilisolol, Mepindolol, Nadolol, Oxprenolol, Acebutolol, Sotalol, Timolol, Labetalol, Penbutolol, Celiprolol, Amosulalol, Alprenolol, Cloranolol, Bopindolol, Soquinolol, Arotinolol, Pindolol, Talinolol, Esatenolol, Indenolol, Befunolol, Bevantolol, Bucomolome, Bunitrolol, Butofilolol, Carazolol, Lervonoprolol, Nifenalol, Rescimetol, Bunazosin, Doxazosin, Guanabenz, Guanadrel, Guanfacine, Guanoxabenz, Indoramine, Rilmenidine, Lofexidine, Naftopidil, Prazosin, and mixtures thereof.
r) SELECTIVE CALCIUM CHANNEL BLOCKERS WITH MAINLY VASCULAR EFFECTS
   Amlodipine, Nisoldipine, Nicardipine, Nitrendipine, Felodipine, Anipamil, Zonisamide, Benidipine, Darodipine, Tiapamil, Tetrandrine, Lercanidipine, Gallopamil, Bepridil, Diproteverine, Isradipine, Franidipine, Nivaldipine, Levetiracetam, Nimodipine, Verapamil, Aranidipine, Fasudil, Dotarizine, Lacidipine, Lomerizine, Cilnidipine, Nifedipine, Diltiazem, Palonidipine, Monatepil, Fantofarone, Semotiadil, Efenidipino, Manidipine, Barnidipine, Elgodipine, Pranidipine, Furaldipine, Ciclandelate, and mixtures thereof.
s) AGENTS ACTING ON THE RENIN-ANGIOTENSIN SYSTEM; ACE INHIBITORS
   Enalapril, Ramipril, Quinapril, Captopril, Perindopril, Fosinopril, Trandolapril, Cilazapril, Lisinopril, Spirapril, Moexipril, Delapril, Alacepril, Enalaprilat, Benazepril, Fentiapril, Zofenopril, Fosinoprilat, Utibapril, Temocapril, Ceranapril, Zofenoprilat, Imidapril, and mixtures thereof.
t) ANG IOTENSIN II ANTAGONISTS
   Candesartan, Losartan, Eprosartan, Irbesartan, Valsartan, Tasosartan, Telmisartan, Olmesartan, and mixtures thereof.
u) CHOLESTEROL AND TRIGLYCERIDE REDUCERS
   Atorvastatin, Lovastatin, Eptastatin, Simvastatin, Fluvastatin, Dalvastatin, Itavastatin, Rosuvastatin, Pravastatin, Probucol, Polycosanol, Ciprofibrate, Fenofibrate, Benzafibrate, Clofibrate, Filicol, Gemfibrozil, Benfluorex, Colestyramine, Phytosterols, Acipimox, Binifibrate, Clinofibrate, Colestilan, Diethylaminoethyl Dextran, Colestrol, Etiroxate, Etofibrate, Gugulipid, Meglutol, Melinamide, Nicritrol, Omacor, Pirifibrate, Sorbinicate, Sulodexide, Sultosilic Acid, and mixtures thereof.
v) ESTROGENS; FEMALE CONTRACEPTIVES
   Estradiol, Ethinylestradiol, Norethisterone, and mixtures thereof.
w) DRUGS USED IN BENIGN PROSTATIC HYPERTROPHY
   Pygeum Extract, Alfuzosin, Dutasteride, Finisteride, Oxendolone, Tamsulosin, and mixtures thereof.
x) CALCIUM HOMEOSTASIS; ANTIPARATHYROID HORMONES
   Calcitonin, Elcatonin, and mixtures thereof.
y) ANTINEOPLASTIC AGENTS
   Ameticine, Atrimustine, Diaziquone, Spiromustine, Melphalan, Elmustine, Estramustine, Ranimustine, Dibromomulcitol, Tauromustine, Temozolomide, Carboplatin, Fotemustine, Aranose, Perfosfamide, Eptaplatin, Busulfan, Porfiromycin, Ifosfamide, Clorambucil, Altretamine, Cisplatin, Lomustine, Improsulfan, Mitobronitol, Mitolactol, Nedaplatin, Oxaliplatin, Prednimustine, Temozolomide, Treosuflan, Trofosfamide, Cyclophosphamide, Methotrexate, Butocin, Capecitabine, Carmofur, Cladribine, Cytarabine, Doxifluridine, Enocitabine, Fludarabine, Gemcitabine, Pentostatin, Raltitrexed, Tegafur, Etoposide, Pirarubicin, Aminoglutethimide, Anastrozole, Bicalutamide, Clodronate, Epitiostanol, Exemestane, Fadrozole, Flutamide, Formestane, Fulvestrant, Letrozole, Mepitiostane, Nilutamide, Tamoxifen, Toremifene, Trilostane, Krestin, Lentinan, Picibanil, Procodazole, Sizofiran, Ukrain, Virulizin, Alitretinoin, Amsacrine, Bexarotene, Docetaxel, Irinotecan, Miltefosine, Mitoxantrone, Nitracrine, Bortezomib, Paclitaxel, Porfimer, Razoxane, Sobuzoxane, Teniposide, Topotecan, Vindesine, Vinorelbine, Geftinib, Imatinib, Bleomycin, Megestrol, Lenograstim, and mixtures thereof.
z) ANTIINFLAMMATORY AND ANTIRHEUMATIC PRODUCTS
   Aceclofenac, Diclofenac, Ketorolac, Meloxicam, Naproxen, Piketoprofen, Acemetacin, Alclofenac, Amfenac, Ampiroxicam, Azapropazone, Bufexamac, Butibufen, Carprofen, Chondroitin, Cinmetacin, Clidanac, Dexketoprofen, Diphenpyramide, Droxicam, Emorfazone, Enfenamic Acid, Epirizole, Etersalate, Fenbufen, Fentiazac, Feprazone, Flunoxaprofen, Flurbiprofen, Guaimesal, Ibuproxam, Indometacin, Ketoprofen, Lonazolac, Mabuprofen, Nabumetone, Nimesulide, Oxametacin, Parsalmide, Perisoxal, Piroxicam, Pranoprofen, Proglumetacin, Proquazone, Proticinic acid, Sulindac, Talniflumate, Tolfenamic Acid, Tolmetin, Zaltoprofen, Benzydamine, Etofenamate, Felbinac, Fepradinol, Idocrilamide, Loteprednol, Vessiflex, Glucosaline, Celecoxib, Hyaluronic Acid, Meclofenamate, Piproxen, Tenoxicam, Valdecoxib, Etoricoxib, Rofecoxib, and mixtures thereof.
aa) BISPHOSPHONATES
   Risedronate, Tiludronate, Clodronate, Pamidronate, Etidronate, Alendronate, Zoledronate, Cimadronate, Neridronate, Olpadronate, Minodronate, Ibandronate, and mixtures thereof.
bb) ANALGESICS
   Acetylsalicylic Acid, Paracetamol, Codeine, Dihydrocodeine, Dexibuprofen, Alminoprofen, Carbasalate, Desflurane, Diflunisal, Enflurane, Etomidate, Floctafenine, Fosfosal, Isoflurane, Isonixin, Ketorolac, Lornoxicam, Clonixinate, Midazolam, Mofezolac, Naproxen, Nefopam, Propofol, Rimazolium, Rofecoxib, Ropivacaine, Sevoflurane, Parecoxib, Propacetamol, Zaltoprofen, Acemetacin, Sulindac, Indometacin, Mefenamic Acid, Ketoprofen, Diclofenac, Piroxicam, Flupirtine, Mofezolac, Ibuprofen, Fenoprofen, Flurbiprofen, Amtolmentin, Fepradinol, Celecoxib, Valdecoxib, Etoricoxib, Fluproquazon, Nefopam, Asthaxantin, and mixtures thereof.
cc) ANTIMIGRAINE PREPARATIONS
   Almotriptan, Propofol, Gabapentin, Zonisamide, Lisinopril, Valproate, Pirprofen, Indoramin, Lidocain, Metoprolol, Ergotamine, Cyproheptadine, Propranolol, Pizotifen, Flunarizine, Nadolol, Metergoline, Ketoprofen, Methysergide, Buclizine, Timolol, Tiaspirone, Topiramate, Somatostatin, Etiracetam, Cinnarizine, Dihydroergotamine, Feverfew, Dronabinol, Dotarizine, Lomerizine, Ibuprofen, Sumatriptan, Naratriptan, Donepezil, Zolmatriptan, Naproxen, Rizatriptan, Montelukast, Frovatriptan, Botulinum Toxin, Alniditan, Avitriptan, Eletriptan, Metoclopramide, Targinine, Aminophylline, Tolfenamic Acid, Isometheptene, and mixtures thereof.
dd) ANTIEPILEPTICS
   Phenobarbital, Clonazepam, Felbamate, Fosphentoin, Gabapentin, Lamotrigine, Levetiracetam, Oxcarbazepine, Tiagabine, Topiramate, Valproate, Vigabatrin, Zonisamide, Milacemide, Denzimol, Bretazenil, Eterobarb, Diazepam, Chlormethiazole, Clonazepam, Clobazam, Mefobarbital, Mephenytoin, Primidone, Acetazolamide, Valpromide, Ralitoline, Fengabine, Licarbazepine, Lorazepam, Antiepilepsirine, Rufinamide, Zaleplon, Abecamil, Losigamone, Selfotel, Midafotel, Remacemide, Carbamazepine, Ethosuximide, Metsuximide, Retigabine, Valnoctamide, and mixtures thereof.
ee) ANTIPSYCHOYTICS
   Haloperidol, Sulpiride, Blonanserin, Spiperone, Rimcazole, Isofloxythepin, Remoxipride, Emonapride, Bretazenil, Zuclopenthixol, Veralipride, Bromperidol, Droperidol, Trifluoperazine, Bromazepam, Levopromazine, Fluopromazine, Perphenazine, Thioridazine, Chlorprothixene, Fluphenazine, Periciazine, Tiotixene, Flupentixol, Benperidol, Fluspirilene, Pimozide, Clozapine, Pipotiazine, Loxapine, Tiapride, Zotepine, Sultopride, Lithium Carbonate, Asenapine, Tiaspirone, Ritanserin, Tandospirone, Amperozide, Clospipramine, Nalmefene, Prochlorperazine, Amisulpride, Levosulpriride, Risperidone, Promazine, Perospirone, Aripiprazole, Chlorpromazine, Carpipramine, Iloperidone, Remoxepride, Carbamazepine, Olanzapine, Quetiapine, Ziprasidone, Valproate, Azaperone, Cyamemazine, Timiperone, Bifeprunox, and mixtures thereof.
ff) ANXIOLYTICS
   Diazepam, Clorazepate, Pyridoxine, Sulpiride, Lorazepam, Phenobarbital, Meprobamate, Buspirone, Suriclone, Citalopram, Brotizolam, Adinazolam, Etizolam, Bretazenil, Medicar, Enciprazine, Loflazepate, Propranolol, Chlordiazepoxide, Hydroxyzine, Trifluoperazine, Oxazepam, Medazepam, Clonazepam, Oxprenolol, Bromazepam, Clobazam, Nordazepam, Ketazolam, Halazepam, Alprozolam, Fluphenazine, Chlorimipramine, Venlafaxine, Ritanserin, Ipsapirone, Tandospirone, Buspirone, Pazinaclone, Flesinoxan, Fluoxetine, Selfotel, Zatosetron, Pagoclone, Carpipramine, Sunepitron, Sertraline, Paroxetine, Cyclobenzaprine, Cyamemazine, Valnoctamide, Clotiazepam, and mixtures thereof.
gg) ANTIDEPRESSANTS
   Citalopram, Venlafaxine, Atomoxetine, Clopradone, Binedaline, Sertraline, Femoxetine, Oxaprotiline, Viqualine, Clovoxamine, Milacemide, Brofaromine, Cianopramine, Moclobemide, Midalcipran, Adinazolam, Nefazodone, Azamianserin, Reboxetine, Tianeptine, Toloxatone, Fluvoxamine, Amitriptyline, Imipramine, Trifluoperazine, Phenelzine, Fluphenazine, Flupentixol, Isocarboxazid, Tranylcypromine, Trimipramine, Desipramine, Opipramol, Nortriptyline, Protriptyline, Doxepin, Lithium Carbonate, Chlorimipramine, Dosulepin, Trazodone, Butriptyline, Viloxazine, Maprotiline, Amoxapine, Lofepramine, Bupropion, Ritanserin, Doconexent, Paroxetine, Ipsapirone, Fengabine, Tandospirone, Setiptiline, Amfebutamone, Lazabemide, Flesinoxan, Adrafinil, Ademetionine, Modafinil, Litoxetine, Fluoxetine, Ceronapril, Cericlamine, Beloxepin, Sunepitron, Agomelatine, Aprepitant, Amineptine, Nomifensine, Chromium Picolinate, and mixtures thereof.
hh) TREATMENT OF ALCOHOL DEPENDANCE
   Acamprosate, Vigabatrin, Diazepam, Disulfiram, Ritanserin, Naltrexon, Nalmefene, Carbamazepine, Hydroxybutyrate, Nitrefazole, Metadoxine, and mixtures thereof.
ii) NASAL DECONGESTANTS
   Pseudoephedrine, Fluticasone, Indanazoline, Tinazoline, Ipratropium, and mixtures thereof.
jj) DRUGS FOR ASTHMA/OBSTRUCTIVE AIRWAYS DISEASES
   Salmeterol, Fenoterol, Ipratropium, Fluticasone, Beclometasone, Flutropium, Talniflumate, Terbutaline, Oxitropium, Rolipram, Seratrodast, Pranlukast, Formoterol, Albuterol, Salbutamol, Midesteine, Tiotropium, Sibenadet, Roflumilast, Aminophylline, Budesonide, Almitrine, Glycopyrrolate, Bambuterol, Mabuterol, Procaterol, Tulobuterol, Rimiterol, Reproterol, Pirbuterol, Daltroban, Ramatroban, Tomelukast, Ibudilast, Pobilukast, Zafirlukast, Montelukast, Methylprednisotone, Dexamethasone, Triamcinolone, Tipredane, Mometasone, Loteprednol, Flunisolide, Hydrocortisone and mixtures thereof.
kk) EXPECTORANTS OR COUGH SUPPRESSANTS
   Carbocisteine, Citiolone, Dropropizine, Cloperastine, Ozagrel, Nesosteine, Levodropropizine, Cistinexine, Dextromethorphan, Guaimesal, Nepinalone, Fudosteine, Quinidine, Hydrocodone, Noscapine, Chlorpheniramine and mixtures thereof.
ll) ANTIHISTAMINES FOR SYSTEMIC USE
   Terfenadine, Ebastine, Dexchlorpheniramine, Azelastine, Acrivastine, Emedastine, Loratadine, Picumast, Diphenhydramine, Promethazine, Fenclozine, Levocabastine, Desloratadine, Cinnarizine, Setastine, Tagorizine, Mizolastine, Cetirizine, Tazifylline, Epinastine, Olopatadine, Bepotastine, Rupatadine, Norastemizol, Triprolidine, Fexofenadine, Ketotifen, Azatadine, Clemastine, Brompheniramine, and mixtures thereof.
mm) BUCAL ANTISEPTICS
   Chlorhexidine, Chloramine-T, Benzalkonium Chloride, and mixtures thereof.
nn) OTHERS
   Sulfamethoxazole, Centella, Calcium Folinate, Palmidrol, Thiomucase, Glucomannan, Leucocianidol, Bacterial Lysate, Spagul, and mixtures thereof.

It is preferred that active substance which can be present in the compositions according to the invention is selected from the group consisting of non-lipophilic active substances. The preferred pharmaceutically active substances are antacid compounds. The preferred antacids for use in the invention are generally carbonate or hydroxycarbonate salts of calcium, magnesium, aluminium, or bismuth and combinations thereof, and are generally very water insoluble. Other antacids such as sodium bicarbonate, calcium bicarbonate, and other carbonates, silicates, and phosphates are included in this invention. Preferred antacids are aluminium and magnesium antacids, such as, for example, aluminium hydroxide and magnesium hydroxide and also preferred are crystalline aluminium magnesium hydroxycarbonates or sulphates such as hydrotalcite, magaldrate and almagate. Almagate is particularly preferred. Mixtures of antacid compounds may be used if desired. When antiacids are used as pharmaceutically active substances they are present in amounts ranging from 5 to 50% by weight of the composition, preferably, between 10 and 45% by weight of the composition, more preferably between 20 and 35% by weight of the composition.

The compositions of the present invention preferably comprise water, more preferably at least 1 % wt. water, and do not comprise edible gums.

The present invention relates also to a process for producing non-tabletted, individually dosed administration forms comprising the following steps:
- forming a composition comprising at least one pharmaceutically active substance dispersed or dissolved within a matrix material comprising a mixture comprising at least 0.2% by weight of a gelatine, based on the composition, an amount greater than 1 % by weight of the composition of at least one stabilising agent, and at least one water-soluble alcohol and/or water as a solvent, which is plastic at elevated temperature and, keeping such composition above 37° C in a heating tank
- transferring the composition, when it is fluid into a heated dosing apparatus
- discharging the composition onto a shaped substrate, through a controlled mechanism so that a constant quantity of the fluid formulation material is thereby dosed onto the substrate
- cooling the composition
- optionally sealing the substrate containing the composition
wherein water is present in an amount not greater than 46% by weight of the composition and the at least one stabilising agent is selected from the group consisting of (i) esters of glycerine and fatty acids; (ii) products resulting from the alcoholysis / esterification reaction of such esters with polyethyleneglycols, and has a melting point in the range of 42° C to 63 °C.

It is an optional embodiment of the present invention that an adhesion-reducing separating agent is placed on the inner surface of a cavity or a blister prior to step (c) of the above-mentioned process. Examples of such adhesion-reducing separating agents are lecithin, talc, starch, vaseline, and fats which are fluid at 25°C.

It is also a preferred embodiment of the present invention that the cavities or blister of the individually dosed administration forms are made of a material selected from PVC (polyvinyl chloride), PVDC (polyvinylidene chloride), PP (polypropylene), Aclar or laminates such as OPA-Aluminium-PVC (oriented polyamide-aluminium-polyvinyl chloride). PVC is particularly preferred.(in full)

The manufacturing processes described and claimed in European patent application number 0 250 578, which are explicitly incorporated by reference, are modified by the addition of the stabilising agent to the composition to be processed and constitute in this modified form particular preferred embodiments of the process under the present invention.

In another aspect the present invention relates to the use of at least one stabilising agent selected from the group consisting of (i) esters of glycerine and fatty acids (ii) products resulting from the alcoholysis / esterification reaction of such esters with polyethyleneglycols, and having a melting point in the range of 42° C to 63° C to facilitate the removal from the blisters or cavities where they have been packaged, of compositions comprising pharmaceutically active substances dispersed or dissolved within a matrix material comprising a mixture of gelatine and at least one water-soluble alcohol and/or water as a solvent, which composition is plastic at elevated temperature.

As used herein the term "plastic at elevated temperature" is meant to designate a composition which can be molded at temperatures comprised between 45°C and 120°C and keeps its molded shape after it cools to 20°C.

As used herein "melting point" is meant to designate the temperature at which the very last visible particle of a small substance's column introduced in a capillary melts as described in the European Pharmacopea 2.2.14. A suitable apparatus for this determination is the Melting Point Apparatus B-540 available from Büchi Labortechnik AG.

As used herein the term "non-tabletted administration form" is intended to mean any form which has not been manufactured by using conventional tabletting processes such as the tabletting of granular or powdery compositions in an excentric or rotary press machine.

As used herein the term "edible gum" is intended to mean polysaccharide gums comprising among others gum arabic, gum tragacanth, agar agar, xanthan gum, alginates.

As used herein the term "water-soluble alcohol" is meant to designate a pharmaceutically acceptable, liquid monohydric or polyhydric alcohol which can be mixed with water to form a uniform solution in a quantity of at least 10 volumes of alcohol per 100 volumes-of water. Examples of such alcohols are ethanol, n-propanol, iso-propanol, glycerol, propylene glycol, 1,3-butylene glycol and polyethylene glycols having a molecular weight comprised between 100 and 600 Dalton.

### REMOVAL FROM BLISTER TEST

The compositions to be tested are manufactured according to the process described in example 1 and dosed into cylindrical cavities of circular cross-section having a diameter of 25 mm of a blister packaging made of PVC. The blister is thermo-sealed with an aluminium foil.

The blisters are then stored in a climatic chamber at 40°C and 75% relative humidity for 10 weeks. After this period they are left at 25°C and 60% relative humidity for 24 hours.

For each product to be tested a panel consisting of 5 expert panellists is given 5 samples of the formulation each, and the panellists are asked to remove the composition from the blister where it is packaged by pressing with the thumb on the plastic wall of the cavity until the composition is expelled from the cavity through the aluminium foil. After the composition has been expelled the remaining aluminium sealing film is removed and the plastic cavity is visually inspected. The panellist are asked to give a sample the rating "Failed" if residues exceeding 0,5 mm in any dimension can be seen in the empty cavity. Otherwise the rating "Passed" must be assigned.

### EXAMPLES

### Example 1

2060,8 g of a 85% solution of glycerine in water are heated in an Erweka SG3W reactor to 65-75 °C. 288 g of pig skin gelatine of 240 degrees Bloom are slowly and continuously added during approximately 4 minutes until complete solubilisation has taken place. The mixture is stirred for 10 additional minutes. 48 g of lecithin are incorporated and the mixture stirred for 10 minutes. 800 g of almagate are then slowly and continuously added during approximately 15 minutes and the mixture stirred for 20 additional minutes at 75-80°C. 3,2 gr of flavour are successively incorporated and the solution stirred for 5 minutes. 4 g of the molten composition are dosed into the cylindrical cavities of circular cross-section having a diameter of 25 mm of a blister packaging made of PVC. The blister is thermo-sealed with an aluminum foil.

The composition of each individual cavity is as follows:

| Ingredient | % wt. |
|---|---|
| Almagate | 25,00 |
| Gelatine | 9,00 |
| Glycerine (100%) | 54,74 |
| Water | 9,66 |
| Lecithin | 1,50 |
| Flavour | 0,10 |

### Examples 2 to 7

Compositions 2 to 7 were manufactured following the process described in example 1 modified in that 1900,8 gr of the glycerine solution were used, and in that 160 g. of a stabilising agent were added after the complete solubilisation of gelatine had taken place and before the addition of lecithin. After the solubilisation of gelatine the mixture was stirred for 20 minutes and the temperature of the reactor was raised to 75-80°C and 160 g of the stabilising agent were slowly and continuously added during approximately 5 minutes.

The following compositions were manufactured following this process:

| Example | Stabilising agent (Tradename) | Stabilising agent (Chemical nature) | Melting range (°C) |
|---|---|---|---|
| 2 | Cutine HR | Hydrogenated castor oil | 87-88 |
| 3 | Compritol 888 ATO | Glyceryl behenate | 71,4-72,2 |
| 4 | Akofine NF | Hydrogenated cottonseed oil | 63,4-53,9 |
| 5 | Estol 3745 GDS T2 | Glyceryl diestearate 80 | 59,0-59,7 |
| 6 | Gelucire 50/13 | Stearoyl macrogol-32 glycerides | 50,3-51,0 |
| 7 | Gelucire 44/14 | Lauryl macrogol-32 glycerides | 43,6-44,2 |

To evaluate the contribution of the stabilising agent, the compositions of examples 1 to 7 were subjected to the "removal from blister test" described above with the following results:

| Example | Removal from Blister Test |
|---|---|
| 1 | Failed |
| 2 | Failed |
| 3 | Failed |
| 4 | Failed |
| 5 | Passed |
| 6 | Passed |
| 7 | Passed |

## Claims

1. A non-tabletted, chewable, individually dosed administration form packaged in a blister or cavity shaped from a film, which administration form is a composition of at least one pharmaceutically active substance dissolved or dispersed within a matrix material comprising a mixture of at least 0.2% by weight, based on the composition, of a gelatine, an amount greater than 1% by weight, based on the composition, of at least one stabilising agent, and at least one water-soluble alcohol and/or water as a solvent, which composition is plastic at elevated temperature, **characterised in that**
a) the stabilising agent is selected from the group consisting of (i) esters of glycerine and fatty acids; (ii) products resulting from the alcoholysis / esterification reaction of such esters of glycerine and fatty acids with polyethyleneglycols; and
b) **in that** the stabilising agent has a melting point in the range of 42° C to 63° C
c) **in that** water is present in an amount not greater than 46% by weight of the composition.

2. An administration form according to claim 1, **characterised in that** it comprises more than 18% by weight of at least one pharmaceutically active substance.

3. An administration form according to claim 1 or 2, **characterised in that** it comprises an antacid.

4. An administration form according to any preceding claim **characterised in that** it comprises at least 10% by weight, based on the composition, of at least one water-soluble alcohol and/or water as a solvent.

5. An administration form according to claim 4 **characterised in that** it comprises more than 46% by weight, based on of the composition, of at least one water-soluble alcohol.

6. An administration form according to any preceding claim, **characterised in that** it comprises water, preferably in an amount exceeding 1 % wt. of the overall composition.

7. An administration form according to any preceding claim **characterised in that** the composition does not comprise edible gums.

8. A process for producing an administration form, comprising the following steps:
• forming a composition comprising at least one pharmaceutically active substance dispersed or dissolved within a matrix material comprising a mixture comprising at least 0.2% by weight of a gelatine, based on the composition, an amount greater than 1 % by weight of the composition of at least one stabilising agent, and at least one water-soluble alcohol and/or water as a solvent, which is plastic at elevated temperature and, keeping such composition above 37° C in a heating tank
• transferring the composition, when it is fluid into a heated dosing apparatus
• discharging the composition onto a shaped substrate, through a controlled mechanism so that a constant quantity of the fluid formulation material is thereby dosed onto the substrate
• cooling the composition
• optionally sealing the substrate containing the composition
wherein water is present in an amount not greater than 46% by weight of the composition and the at least one stabilising agent is selected from the group consisting of (i) esters of glycerine and fatty acids; (ii) products resulting from the alcoholysis / esterification reaction of such esters with polyethyleneglycols, and has a melting point in the range of 42° C to 63 °C.

9. A process according to claim 8 **characterised in that** the composition comprises more than 18% by weight of a pharmaceutically active substance.

10. A process according to claim 8 or 9 **characterised in that** the pharmaceutically active substance is an antacid.

11. A process according to any one of claims 8 to 10 **characterised in that** the composition comprises water, preferably in an amount exceeding 1% wt. of the overall composition.

12. A process according to any one of claims 8 to 11 **characterised in that** the composition comprises at least 10% by weight of the composition of at least one water-soluble alcohol and/or water as a solvent.

13. A process according to any one of claims 8 to 12 **characterised in that** the composition comprises more than 46% by weight of the composition of at least one water-soluble alcohol.

14. A process according to any one of claims 8 to 13 **characterised in that** the composition does not comprise edible gums.

15. A process according to anyone of claims 8 to 16, wherein the substrate comprises a material selected from the group consisting of PVC, PVDC, PP, Aclar or laminates such as OPA-Aluminium-PVC.

16. Non-tabletted, chewable, individually dosed administration forms obtainable by the process of claims 8 to 17.

17. Use of at least one stabilising agent selected from the group consisting of (i) esters of glycerine and fatty acids (ii) products resulting from the alcoholysis / esterification reaction of such esters with polyethyleneglycols, having a melting point in the range of 42° C to 63° C to facilitate the removal from the blisters or cavities where they have been packaged, of compositions comprising at least one pharmaceutically active substance dispersed or dissolved within a matrix material comprising a mixture of gelatine and at least one water-soluble alcohol and/or water as a solvent, which composition is plastic at elevated temperature.

18. Use according to claim 17 wherein the stabilising agent is used in an amount greater than 1% by weight of the composition.

## Patentansprüche

1. Nicht-tablettierte, kaubare, individuell dosierte Verabreichungsform, die in einem Blister oder einer Kavität, geformt aus einem Film, verpackt ist, wobei die Verabreichungsform eine Zusammensetzung wenigstens einer pharmazeutisch aktiven Substanz ist, die in einem Matrixmaterial gelöst oder dispergiert ist, welches ein Gemisch aus wenigstens 0,2 Gew.-%, bezogen auf die Zusammensetzung, einer Gelatine, einer Menge von größer als 1 Gew.-%, bezogen auf die Zusammensetzung, wenigstens eines Stabilisierungsmittels und wenigstens einem wasserlöslichen Alkohol und/oder Wasser als Lösungsmittel umfasst, wobei die Zusammensetzung bei erhöhter Temperatur plastisch ist, **dadurch gekennzeichnet, dass**
a) das Stabilisierungsmittel aus der Gruppe, bestehend aus (i) Estern von Glycerin und Fettsäuren; (ii) Produkten, die aus der Alkoholyse/Veresterungs-Reaktion solcher Ester von Glycerin und Fettsäuren mit Polyethylenglykolen resultieren, ausgewählt ist, und
b) dass das Stabilisierungsmittel einen Schmelzpunkt im Bereich von 42°C bis 63°C hat,
c) das Wasser in einer Menge von nicht größer als 46 Gew.-% der Zusammensetzung vorliegt.

2. Verabreichungsform nach Anspruch 1, **dadurch gekennzeichnet, dass** sie mehr als 18 Gew.-% wenigstens einer pharmazeutisch aktiven Substanz umfasst.

3. Verabreichungsform nach Anspruch 1 oder 2, **dadurch gekennzeichnet, dass** sie ein Antacidum umfasst.

4. Verabreichungsform nach einem vorangehenden Anspruch, **dadurch gekennzeichnet, dass** sie wenigstens 10 Gew.-%, bezogen auf die Zusammensetzung, wenigstens eines wasserlöslichen Alkohols und/oder Wasser als Lösungsmittel umfasst.

5. Verabreichungsform nach Anspruch 4, **dadurch gekennzeichnet, dass** sie mehr als 46 Gew.-%, bezogen auf die Zusammensetzung, wenigstens eines wasserlöslichen Alkohols umfasst.

6. Verabreichungsform nach einem vorangehenden Anspruch, **dadurch gekennzeichnet, dass** sie Wasser, vorzugsweise in einer Menge, die 1 Gew.-% der Gesamtzusammensetzung übersteigt, umfasst.

7. Verabreichungsform nach einem vorangehenden Anspruch, **dadurch gekennzeichnet, dass** die Zusammensetzung keine essbaren Gummen umfasst.

8. Verfahren zur Herstellung einer Verabreichungsform, umfassend die folgenden Schritte:
• Bilden einer Zusammensetzung, umfassend wenigstens eine pharmazeutisch aktive Substanz, dispergiert oder gelöst in einem Matrixmaterial, welches ein Gemisch umfasst, das wenigstens 0,2 Gew.-% einer Gelatine, bezogen auf die Zusammensetzung, eine Menge von größer als 1 Gew.-%, bezogen auf die Zusammensetzung, wenigstens eines Stabilisierungsmittels und wenigstens einen wasserlöslichen Alkohol und/oder Wasser als Lösungsmittel umfasst, welche bei erhöhter Temperatur plastisch ist, und Halten einer derartigen Zusammensetzung bei über 37°C in einem Wärmetank;
• Transferieren der Zusammensetzung, wenn sie flüssig ist, in eine erwärmte Dosierapparatur;
• Austragen der Zusammensetzung auf ein geformtes Substrat durch einen gesteuerten Mechanismus derart, dass eine konstante Menge des flüssigen Formulierungsmaterials **dadurch** auf das Substrat dosiert wird;
• Kühlen der Zusammensetzung;
• gegebenenfalls Versiegeln des Substrats, das die Zusammensetzung enthält;
wobei Wasser in einer Menge von nicht größer als 46 Gew.-% der Zusammensetzung vorliegt und das wenigstens eine Stabilisierungsmittel aus der Gruppe, bestehend aus (i) Estern von Glycerin und Fettsäuren; (ii) Produkten, die aus der Alkoholyse/Veresterungs-Reaktion solcher Ester mit Polyethylenglykolen resultieren, ausgewählt ist und einen Schmelzpunkt im Bereich von 42°C bis 63°C hat.

9. Verfahren nach Anspruch 8, **dadurch gekennzeichnet, dass** die Zusammensetzung mehr als 18 Gew.-% einer pharmazeutisch aktiven Substanz umfasst.

10. Verfahren nach Anspruch 8 oder 9, **dadurch gekennzeichnet, dass** die pharmazeutisch aktive Substanz ein Antacidum ist.

11. Verfahren nach einem der Ansprüche 8 bis 10, **dadurch gekennzeichnet, dass** die Zusammensetzung Wasser, vorzugsweise in einer Menge, die 1 Gew.-% der Gesamtzusammensetzung übersteigt, umfasst.

12. Verfahren nach einem der Ansprüche 8 bis 11, **dadurch gekennzeichnet, dass** die Zusammensetzung wenigstens 10 Gew.-%, bezogen auf die Zusammensetzung, wenigstens eines wasserlöslichen Alkohols und/oder Wasser als Lösungsmittel umfasst.

13. Verfahren nach einem der Ansprüche 8 bis 12, **dadurch gekennzeichnet, dass** die Zusammensetzung mehr als 46 Gew.-%, bezogen auf die Zusammensetzung, wenigstens eines wasserlöslichen Alkohols umfasst.

14. Verfahren nach einem der Ansprüche 8 bis 13, **dadurch gekennzeichnet, dass** die Zusammensetzung keine essbaren Gummen umfasst.

15. Verfahren nach einem der Ansprüche 8 bis 16, wobei das Substrat ein Material umfasst, das aus der Gruppe, bestehend aus PVC, PVDC, PP, Aclar oder Laminaten, zum Beispiel OPA-Aluminium-PVC, ausgewählt ist.

16. Nicht-tablettierte, kaubare, individuell dosierte Verabreichungsformen, erhältlich durch das Verfahren nach den Ansprüchen 8 bis 17.

17. Verwendung wenigstens eines Stabilisierungsmittels, das aus der Gruppe, bestehend aus (i) Estern von Glycerin und Fettsäuren, (ii) Produkten, die aus der Alkoholyse/Veresterungs-Reaktion solcher Ester mit Polyethylenglykolen resultieren, ausgewählt ist, das einen Schmelzpunkt im Bereich von 42°C bis 63°C hat, um die Entfernung von Zusammensetzungen, die wenigstens eine pharmazeutisch aktive Substanz, dispergiert oder gelöst innerhalb eines Matrixmaterials, das ein Gemisch aus Gelatine und wenigstens einem wasserlöslichen Alkohol und/oder Wasser als Lösungsmittel umfasst, umfassen, aus den Blistern oder Kavitäten, in die sie verpackt wurden, zu erleichtern, wobei die Zusammensetzung bei erhöhter Temperatur plastisch ist.

18. Verwendung nach Anspruch 17, wobei das Stabilisierungsmittel in einer Menge von größer als 1 Gew.-%, bezogen auf die Zusammensetzung, verwendet wird.

## Revendications

1. Forme d'administration dosée individuellement, masticable, n'étant pas sous la forme d'un comprimé, conditionnée dans un blister ou une cavité façonnés à partir d'un film, laquelle forme d'administration est une composition d'au moins une substance pharmaceutiquement active dissoute ou dispersée à l'intérieur d'un matériau de matrice comprenant un mélange d'au moins 0,2 % en poids, par rapport à la composition, d'une gélatine, d'une quantité supérieure à 1 % en poids, par rapport à la composition, d'au moins un agent stabilisant, et d'au moins un alcool hydrosoluble et/ou de l'eau en tant que solvant, laquelle composition est plastique à température élevée, **caractérisée en ce que** :
a) l'agent stabilisant est choisi dans le groupe constitué par (i) les esters de glycérine et d'acides gras ; (ii) les produits résultant de la réaction d'alcoolyse/estérification de tels esters de glycérine et d'acides gras avec des polyéthylèneglycols ; et
b) l'agent stabilisant a un point de fusion dans la gamme de 42 °C à 63 °C
c) l'eau est présente en une quantité non supérieure à 46 % en poids de la composition.

2. Forme d'administration selon la revendication 1, **caractérisée en ce qu'**elle comprend plus de 18 % en poids d'au moins une substance pharmaceutiquement active.

3. Forme d'administration selon la revendication 1 ou 2, **caractérisée en ce qu'**elle comprend un antiacide.

4. Forme d'administration selon l'une quelconque des revendications précédentes, **caractérisée en ce qu'**elle comprend au moins 10 % en poids, par rapport à la composition, d'au moins un alcool hydrosoluble et/ou d'eau en tant que solvant.

5. Forme d'administration selon la revendication 4, **caractérisée en ce qu'**elle comprend plus de 46 % en poids, par rapport à la composition, d'au moins un alcool hydrosoluble.

6. Forme d'administration selon l'une quelconque des revendications précédentes, **caractérisée en ce qu'**elle comprend de l'eau, de préférence en une quantité dépassant 1 % en poids de l'ensemble de la composition.

7. Forme d'administration selon l'une quelconque des revendications précédentes, **caractérisée en ce que** la composition ne comprend pas de gommes comestibles.

8. Procédé pour produire une forme d'administration, comprenant les étapes suivantes :
• formation d'une composition comprenant au moins une substance pharmaceutiquement active dispersée ou dissoute à l'intérieur d'un matériau de matrice comprenant un mélange d'au moins 0,2 % en poids d'une gélatine, par rapport à la composition, d'une quantité supérieure à 1 % en poids de la composition d'au moins un agent stabilisant, et d'au moins un alcool hydrosoluble et/ou de l'eau en tant que solvant, qui est plastique à température élevée, en maintenant cette composition au-dessus de 37 °C dans un réservoir chauffant
• transfert de la composition, lorsqu'elle est fluide, jusque dans un appareil de dosage chauffé
• décharge de la composition sur un substrat façonné, par l'intermédiaire d'un mécanisme contrôlé de sorte qu'une quantité constante de la matière de formulation fluide est ainsi dosée sur le substrat
• refroidissement de la composition
• éventuellement, scellement du substrat contenant la composition
dans lequel l'eau est présente en une quantité non supérieure à 46 % en poids de la composition et ledit au moins un agent stabilisant est choisi dans le groupe constitué par (i) les esters de glycérine et d'acides gras ; (ii) les produits résultant de la réaction d'alcoolyse/estérification de tels esters avec des polyéthylèneglycols, et a un point de fusion dans la gamme de 42 °C à 63 °C.

9. Procédé selon la revendication 8, **caractérisé en ce que** la composition comprend plus de 18 % en poids d'une substance pharmaceutiquement active.

10. Procédé selon la revendication 8 ou 9, **caractérisé en ce que** la substance pharmaceutiquement active est un antiacide.

11. Procédé selon l'une quelconque des revendications 8 à 10, **caractérisé en ce que** la composition comprend de l'eau, de préférence en une quantité dépassant 1 % en poids de l'ensemble de la composition.

12. Procédé selon l'une quelconque des revendications 8 à 11, **caractérisé en ce que** la composition comprend au moins 10 % en poids de la composition d'au moins un alcool hydrosoluble et/ou d'eau en tant que solvant.

13. Procédé selon l'une quelconque des revendications 8 à 12, **caractérisé en ce que** la composition comprend plus de 46 % en poids de la composition d'au moins un alcool hydrosoluble.

14. Procédé selon l'une quelconque des revendications 8 à 13, **caractérisé en ce que** la composition ne comprend pas de gommes comestibles.

15. Procédé selon l'une quelconque des revendications 8 à 16, dans lequel le substrat comprend un matériau choisi dans le groupe constitué par un PVC, un PVDC, un PP, l'Aclar ou des stratifiés tels que OPA-aluminium-PVC.

16. Formes d'administration dosées individuellement, masticables, n'étant pas sous la forme de comprimé, susceptibles d'être obtenues par le procédé selon les revendications 8 à 17.

17. Utilisation d'au moins un agent stabilisant choisi dans le groupe constitué par (i) les esters de glycérine et d'acides gras (ii) les produits résultant de la réaction d'alcoolyse/estérification de tels esters avec des polyéthylèneglycols, ayant un point de fusion dans la gamme de 42 °C à 63 °C pour faciliter l'enlèvement, à partir des blisters ou des cavités dans lesquels elles sont conditionnées, de compositions comprenant au moins une substance pharmaceutiquement active dispersée ou dissoute à l'intérieur d'un matériau de matrice comprenant un mélange de gélatine et d'au moins un alcool hydrosoluble et/ou d'eau en tant que solvant, ladite composition est plastique à température élevée.

18. Utilisation selon la revendication 17, dans laquelle l'agent stabilisant est utilisé en une quantité supérieure à 1 % en poids de la composition.
